# FASCICULE DE BREVET EUROPEEN

(11) **EP 2 222 634 B1**
(45) Date de publication et mention de la délivrance du brevet: **17.04.2013**
(21) Numéro de dépôt: 08855128.8
(22) Date de dépôt: 25.11.2008
(51) Int. Cl.: C07C 313/04

(54) **PROCEDE DE PREPARATION DE L'ACIDE TRIFLUOROMETHANESULFINIQUE.**
VERFAHREN ZUR HERSTELLUNG VON TRIFLUORMETHANSULFINSÄURE
METHOD FOR PREPARING TRIFLUOROMETHANESULPHINIC ACID

(30) Priorité: 27.11.2007 FR 0708281
(43) Date de publication de la demande: 01.09.2010
(73) Titulaire: Rhodia Opérations, 93306 Aubervilliers (FR)
(72) Inventeur: SCHANEN, Vincent, F-69006 Lyon (FR); BUISINE, Olivier, F-69230 Saint Genis Laval (FR); METZ, Bernard, F-69540 Irigny (FR); BESSON, Bernard, F-01700 Les Echets (FR)
(74) Mandataire: Dutruc-Rosset, Marie-Claude
(86) Numéro de dépôt international: PCT/EP2008/066161
(87) Numéro de publication internationale: WO 2009/068533

(56) Documents cités:
- EP-A- 0 735 023
- WO-A-01/49659
- C. HARZDORF, ET AL.: "Über Perfluoralkansulfinsäuren" LIEBIGS ANNALEN DER CHEMIE, no. 1, 22 février 1973 (1973-02-22), pages 33-39, XP002491378 VERLAG CHEMIE, WEINHEIM, DE

## Description

La présente invention a pour objet un procédé de préparation de l'acide trifluorométhanesulfinique.

L'invention vise la préparation d'un acide trifluorométhanesulfinique de haute pureté.

L'acide trifluorométhanesulfinique dénommé couramment « acide triflinique » ou bien ses formes salifiées sont des produits utilisés dans de nombreux domaines, phytosanitaire, pharmaceutique ou autre.

Une voie de synthèse dudit acide est décrite dans C. Harzdorf, et al., Liebigs Annalen der Chemie 1973, (1), 33-39. Elle consiste à faire réagir le fluorure de trifluorométhanesulfonyle avec de l'hydrate d'hydrazine dans un solvant organique polaire.

Une autre des voies de synthèse dudit acide décrite dans EP 0 735 023, consiste à faire réagir l'acide trifluoroacétique, au moins partiellement salifié par un cation organique ou minéral avec du dioxyde de soufre dans un solvant organique polaire et chauffage du mélange résultant à une température comprise entre 100°C et 200°C pendant une durée comprise entre 30 min et 20 heures.

Les quantités relatives de l'acide trifluoroacétique et du dioxyde de soufre sont telles que le rapport entre le nombre d'atomes de soufre par mole d'acide trifluoroacétique est compris entre 1 et 10, avantageusement aux alentours de deux.

En fin de réaction, on obtient l'acide trifluoroacétique, l'acide trifluorométhanesulfinique sous forme saline, de préférence sous forme d'un sel de métal alcalin, préférentiellement le sodium ou le potassium et le solvant organique.

Sont également coproduits au cours de la réaction des sels, fluorure ou sulfate, le plus souvent sous forme de sel d'un métal alcalin, de préférence le sodium ou le potassium. Ces sels formés sont dénommés « impuretés salines » dans la suite du présent texte. Il est déjà connu de procéder au traitement d'un effluent de ce type contenant du trifluorométhanesulfinate de potassium, du trifluoroacétate de potassium et du diméthylformamide pour récupérer un solide essentiellement à base de trifluorométhanesulfinate de potassium (WO 2001/49659).

A l'issue de la réaction en présence de dioxyde de soufre décrite ci-dessus, on effectue ensuite une dilution par addition d'eau puis l'on extrait le solvant organique polaire par un solvant organique approprié, par exemple un hydrocarbure aliphatique chloré.

On sépare les phases organique et aqueuse.

On obtient généralement une solution aqueuse ayant une teneur en matières sèches de 10 à 40 % en masse comprenant :
- de 5 à 35 % en masse d'un sel d'acide trifluorométhanesulfinique, de préférence un sel alcalin,
- de 5 à 35 % en masse d'un sel d'acide trifluoroacétique, de préférence un sel alcalin,
- de 0,5 à 2 % en masse d'impuretés salines.

Ladite solution aqueuse comprend préférentiellement de 15 à 20 % en masse d'un sel d'acide trifluorométhanesulfinique, de 10 à 15 % en masse d'un sel d'acide trifluoroacétique et de 0,5 à 2 % en masse d'impuretés salines.

L'objectif de la présente invention est de fournir un acide trifluorométhanesulfinique de haute pureté à partir d'une phase aqueuse comprenant un sel d'acide trifluorométhanesulfinique associé à un sel d'acide trifluoroacétique et des impuretés salines résultant de son procédé de préparation.

Par « haute pureté », on entend dans le présent texte, un acide trifluorométhanesulfinique ayant une pureté supérieure ou égale à 95 %, de préférence supérieure ou égale à 98 % et plus préférentiellement supérieure ou égale à 99 %.

Ainsi, la présente invention a pour objet un procédé de préparation d'un acide trifluorométhanesulfinique de haute pureté, à partir d'un mélange aqueux comprenant un sel d'acide trifluorométhanesulfinique, un sel d'acide trifluoroacétique et des impuretés salines résultant de son procédé de préparation, caractérisé par le fait que ledit mélange est soumis aux opérations suivantes :
- acidification de telle sorte que l'on libère les sels de l'acide trifluoroacétique et de l'acide triflinique sous une forme acide,
- séparation des acides trifluoroacétique et trifluorométhanesulfinique par distillation permettant de récupérer l'acide trifluoroacétique en tête de distillation et l'acide trifluorométhanesulfinique en pied de distillation, séparation par distillation de l'acide trifluorométhanésulfinique présent dans le culot de distillation précédemment obtenu.

Dans la suite de l'exposé de l'invention, l'acide trifluorométhanesulfinique est désigné « acide triflinique ».

Le procédé de l'invention est fondé sur le fait que l'on effectue une acidification de telle sorte que l'on libère les sels de l'acide trifluoroacétique et de l'acide triflinique sous une forme acide puis que l'on sépare lesdits acides obtenus par distillation.

Selon une variante préférée du procédé de l'invention, on soumet le mélange aqueux recueilli à une opération de concentration.

En effet, il est souhaitable d'effectuer avant l'opération d'acidification, une concentration dudit mélange aqueux.

A cet effet, on peut effectuer une concentration du mélange aqueux précédemment défini de façon à augmenter la concentration des sels des acides trifluoroacétique et triflinique de telle sorte qu'il y ait présence entre 2 et 60 % en masse, de préférence entre 5 et 50 % en masse d'eau dans la solution concentrée obtenue.

Un mode de réalisation pour concentrer le milieu réactionnel consiste à effectuer la distillation d'une partie de l'eau pour atteindre dans le milieu réactionnel la concentration en eau souhaitée.

On peut effectuer la distillation sous pression atmosphérique à une température de 100°C.

On peut également effectuer la distillation sous une pression légèrement inférieure à la pression atmosphérique, par exemple comprise entre 1 mbar et 600 mbar et à une température inférieure à 100°C. En général, la pression est choisie pour avoir une température de distillation située entre 40°C et 90°C.

Un autre mode consiste à effectuer un entraînement par injection d'un fluide, par exemple vapeur ou gaz inerte, notamment azote.

D'un point de vue pratique, les opérations de concentration de même que les opérations de distillation décrites ci-après peuvent être conduites dans un évaporateur. On peut utiliser ceux qui sont disponibles dans le commerce et l'on peut citer entre autres, les évaporateurs à film raclé ou tombant de type LUWA^{®}_{.}

L'invention n'exclut pas la mise en oeuvre d'autres techniques de concentration telles que l'ultrafiltration ou l'osmose inverse.

Conformément au procédé de l'invention, on effectue ensuite une acidification de la solution aqueuse concentrée.

L'acidification est réalisée à l'aide d'un acide fort ayant un pkₐ inférieur ou égal à 1.

Le pKa est défini comme la constante de dissociation ionique du couple acide/base, lorsque l'eau est utilisée comme solvant.

On choisit un acide fort qui avantageusement ne présente pas de caractère oxydant. Ainsi, l'acide nitrique n'est pas préféré. On fait appel plus préférentiellement à l'acide sulfurique, chlorhydrique ou phosphorique.

L'acide sulfurique est choisi préférentiellement.

La quantité d'acide fort mis en oeuvre est telle que le rapport entre le nombre de moles d'acide exprimé en ions H⁺ et le nombre de moles des sels d'acide trifluoroacétique et d'acide triflinique varie entre 1 et 10, de préférence entre 1 et 4.

On fait appel avantageusement à une solution d'acide fort concentrée.

On utilise plus particulièrement les formes commerciales d'acides.

On peut citer notamment les solutions d'acide sulfurique à 95 ou 98 % en masse, d'acide chlorhydrique à 37 % en masse, d'acide phosphorique à 95 - 100 % en masse.

On peut également utiliser l'acide chlorhydrique sous forme gazeuse ou des oléums qui correspondent à l'acide sulfurique chargé d'anhydride sulfurique SO₃ dont la concentration peut varier entre 10 % et 60 % en masse. Des oléums à 20 %, 40 % ou 60 % en masse de SO₃ sont commercialement disponibles.

En fin de traitement acide, on recueille une solution ou une suspension aqueuse comprenant l'acide trifluoroacétique, l'acide triflinique, l'acide fort en excès, les sels formés suite à l'acidification et les formes acides correspondant aux impuretés salines.

Généralement, la solution ou la suspension obtenue comprend de 4 à 26 % en masse d'acide trifluoroacétique, de 4 à 27 % en masse d'acide triflinique et de 5 à 60 % en masse d'eau.

Selon une variante de l'invention, il est possible d'ajouter dans la solution ou la suspension aqueuse obtenue suite à la concentration mais avant l'acidification, un composé susceptible de piéger l'acide fluorhydrique, par exemple l'acide borique ou de la silice, par exemple à une teneur de 1 % en masse.

Avant de procéder à la distillation de l'acide trifluoroacétique, il est possible mais non impératif de procéder à une séparation des solides présents dans la suspension aqueuse (sels, silice etc...) selon les techniques classiques de séparation solide/liquide, de préférence par filtration.

Dans une étape suivante, on procède à une opération de distillation ce qui permet d'effectuer la séparation des acides trifluoroacétique et triflinique.

On introduit la phase aqueuse précitée comprenant les acides à séparer dans une colonne de distillation, et où on élimine en tête de distillation, l'acide trifluoroacétique et éventuellement de l'eau et l'on récupère en pied de distillation l'acide triflinique accompagné de l'acide fort en excès, des sels des acides forts notamment ceux provenant de l'acidification et de l'eau.

La distillation est conduite à une température dans le bouilleur comprise entre 60°C et 90°C sous une pression allant de 700 mbar à 50 mbar.

Elle est effectuée dans un appareil de distillation classique.

L'homme du métier est parfaitement en mesure de choisir les moyens à mettre en oeuvre en fonction des composés à séparer.

On rappellera simplement ce qui suit. La taille (notamment le diamètre) des colonnes de distillation dépend du flux circulant et de la pression interne. Leur dimensionnement se fera donc principalement suivant le débit de mélange à traiter. Le paramètre interne qu'est le nombre d'étages théoriques est déterminé notamment par la pureté du composé de départ et la pureté du produit devant être obtenus en tête de distillation.

On précisera que les colonnes pourront être garnies indifféremment de plateaux ou de garnissage ordonné, comme cela est parfaitement connu de l'homme du métier.

L'installation étant déterminée, l'homme du métier ajuste les paramètres de fonctionnement de la colonne.

Ainsi, la colonne de distillation pourra être avantageusement, mais non limitativement, une colonne ayant les spécifications suivantes :
- nombre d'étages théoriques : de 1 à 10, de préférence de 1 à 5,
- taux de reflux R compris entre 1 et 20 , de préférence entre 5 et 10.

En bas de colonne, on récupère un culot de distillation comprenant l'acide triflinique et en tête de colonne une phase gazeuse constituée par l'acide trifluoroacétique accompagnée éventuellement d'eau.

On refroidit la phase gazeuse et la transforme sous forme liquide par refroidissement à une température par exemple comprise entre -20°C et 20°C, de préférence comprise entre -10°C et 10°C.

Cette opération est conduite par passage dans un condenseur qui est un appareil classique par exemple un échangeur tubulaire alimenté par de l'eau ou par un fluide maintenu à une température voisine de la température de refroidissement choisie.

On choisit avantageusement pour la conduite de l'opération de distillation un appareillage susceptible de résister à la corrosion provoquée par les composés à séparer.

A cet effet, on choisit des matériaux avantageusement des aciers vitrifiés.

On effectue ensuite le traitement du culot de distillation comprenant l'acide triflinique que l'on souhaite obtenir.

A cet effet, on effectue la distillation de l'acide triflinique à une température qui pour des raisons de sécurité est maintenue inférieure à 100°C, de préférence inférieure à 90°C sous pression réduite choisie de préférence inférieure à 50 mbar et comprise plus préférentiellement entre 0,1 mbar et 30 mbar. On choisit avantageusement une pression comprise entre 5 mbar et 30 mbar.

L'invention n'exclut pas l'addition d'un tiers solvant afin de faciliter la distillation. On peut citer en particulier les hydrocarbures aliphatiques comme par exemple le décane, la décaline, les coupes pétrolières commercialisées sous la dénomination ISOPAR ® ; les hydrocarbures aromatiques tels que notamment, le toluène, les xylènes, le mésitylène ; les hydrocarbures aromatiques halogénés et tout particulièrement le monochlorobenzène, le dichlorobenzène ou leurs mélanges.

La quantité de tiers solvant mise en oeuvre exprimée par rapport à la masse présente dans le bouilleur, peut varier assez largement. Elle peut être égale par exemple de 10 à 100 %, et de préférence de 20 à 50 % en masse.

On récupère en tête de distillation l'acide triflinique éventuellement accompagné du tiers solvant qui est liquéfié par passage dans un condenseur maintenu à une température comprise entre - 20°C et 20°C.

On obtient en pied de distillation un culot comprenant de l'acide triflinique résiduaire, les sels des acides forts, l'acide fort en excès utilisé à l'acidification, de l'eau et éventuellement le tiers solvant ajouté.

L'invention n'exclut pas le cas où l'on effectue une distillation supplémentaire après l'opération d'acidification, permettant de recueillir un mélange des acides trifluoroacétique et trifluorométhanesulfinique, puis à partir du mélange obtenu desdits acides, la séparation des acides trifluoroacétique et trifluorométhanesulfinique réalisée comme précédemment décrit à savoir une première distillation permettant de récupérer l'acide trifluoroacétique en tête de distillation et l'acide trifluorométhanesulfinique en pied de distillation, puis ensuite une deuxième distillation du culot de distillation obtenu précédemment permettant de récupérer en tête de distillation, l'acide trifluorométhanesulfinique attendu.

Dans cette variante, les conditions de distillation permettant de récupérer le mélange des acides trifluoroacétique et trifluorométhanesulfinique, sont celles définies pour la récupération de l'acide trifluorométhanesulfinique.

Le procédé de l'invention est très intéressant car il conduit à l'acide triflinique d'une grande pureté, de préférence supérieure ou égale à 95 %, plus préférentiellement supérieure ou égale à 98 % et comprise plus particulièrement entre 99 et 99,5 %.

On donne ci-après des exemples de réalisation de l'invention donnés à titre indicatif et sans caractère limitatif.

### Exemple 1

Une solution aqueuse (774 g) contenant 16 % en masse de trifluorométhanosulfinate de potassium et 13,5 % en masse de trifluoroacétate de potassium est chargée dans un réacteur en verre, à double enveloppe de 1 litre équipé d'une agitation centrale et maintenu sous atmosphère d'azote.

L'ensemble est mis sous une pression variant de 14 mbar à 2 mbar en maintenant une température de 50°C.

On obtient 225 g d'une suspension qui reste fluide à 50°C.

La solution concentrée est coulée sur de l'acide sulfurique (780 g) concentré à 92 % dans un réacteur surmonté d'une colonne de distillation et d'un condenseur refroidi à l'eau (15°C).

La température du milieu est maintenue inférieure à 30°C durant la coulée de la solution aqueuse concentrée.

La pression est abaissée à 280 mbar et la température du bouilleur est maintenue à 68°C.

Au cours de la distillation, la pression est progressivement abaissée à 86 mbar pour maintenir un débit de distillat suffisant.

Une première fraction de distillation est récoltée (56 g) lorsque la température des vapeurs en tête de colonne est comprise entre 26°C et 36°C.

Cette fraction est constituée à 85 % en masse d'acide trifluoroacétique et 15 % d'acide triflinique.

La pression est ensuite abaissée à 3 mbar.

Une seconde fraction (44 g) est récoltée lorsque la température des vapeurs en tête de colonne est comprise entre 29°C et 41 °C.

Durant cette opération, la température du bouilleur varie de 70°C à 79°C.

Cette fraction contient de l'acide trifluorométhanesulfinique pur à 99 %.

### Exemple 2

Une solution aqueuse (3000 g) contenant 16 % en masse de trifluorométhanesulfinate de potassium et 13,5 % en masse de trifluoroacétate de potassium est concentrée sur un évaporateur à film raclé de type LUWA de surface d'échange 314 cm², maintenu à une température de 80°C et une pression de 50 mbar.

Le débit d'alimentation est ajusté de façon à obtenir 980 g de solution concentrée en sortie de l'évaporateur.

La solution concentrée est coulée sur 2940 g d'acide sulfurique concentré à 96 % en maintenant la température inférieure à 20°C.

La pression du réacteur est ajustée à 280 mbar et la température de la masse réactionnelle est portée à 78°C.

Une première fraction de 274 g est récoltée lorsque la température en tête de colonne est comprise entre 41 °C et 45°C.

La pression est ensuite maintenue à 1 mbar et une seconde fraction 245 g est récoltée.

Cette fraction contient de l'acide trifluorométhanesulfinique pur à 99 %.

### Exemple 3

Un mélange constitué de trifluoroacétate de potassium (84,0 g, 0,55 mol), de trifluorométhanesulfinate de potassium (93,4 g, 0,54 mol) et d'eau (44 g, 2,4 mol) est ajouté à de l'acide phosphorique (600 g, 6,1 mol) porté à 60°C et laissé sous agitation pendant une heure.

Le milieu est ensuite distillé sous pression réduite à l'aide d'une colonne de Vigreux.

La température du milieu est portée à 70°C sous pression réduite de150 mbar.

On obtient ainsi une fraction de 62,9 g ayant un point d'ébullition de 24°C à cette pression.

Cette fraction contient 94 % en masse d'acide trifluoroacétique et 6 % en masse d'eau.

Le rendement en acide trifluoroacétique est de 84 %.

La température est ensuite montée à 90°C et la pression est abaissée à 0,3 mbar.

On obtient 71,1 g d'une seconde fraction de distillation ayant un point d'ébullition de 28°C.

Cette fraction contient 79 % en masse d'acide trifluorométhanesulfinique et 21 % en masse d'acide trifluoroacétique.

Le rendement en acide trifluorométhanesulfinique est de 77 %.

Cette fraction est redistillée sous une pression 25 mbar.

On obtient alors une première fraction (16 g) contenant 94 % massique d'eau et une seconde fraction (47,8 g) contenant 98 % en masse d'acide trifluorométhanesulfinique.

### Exemple 4

A une solution aqueuse (364 g) contenant du trifluoroacétate de potassium (95 g, 0,63 mol) et du trifluorométhanesulfinate de potassium (108 g, 0,63 mol) est ajouté de l'acide sulfurique concentré à 95 % massique (162 g, 1,6 mol) en maintenant la température du milieu inférieure à 10°C.

Un solide se forme dans le milieu.

L'agitation du milieu est maintenue 30 minutes puis le solide (205 g) est éliminé par filtration sur un fritté de porosité 3.

Le filtrat obtenu (301 g) est distillé sous pression réduite.

Le milieu est porté à une température de 55°C en masse sous une pression de 18 mbar.

On obtient une première fraction (201 g) ayant un point d'ébullition d'environ 25°C.

Celle-ci contient 27 % en masse d'acide trifluoroacétique et 73 % en masse d'eau.

Le rendement en acide trifluoroacétique est de 77 %.

Le milieu est ensuite porté à une température de 95°C sous une pression de 0,2 mbar.

On obtient une seconde fraction de distillation (57 g) contenant 72 % en masse d'acide trifluorométhanesulfinique et 28 % en masse d'eau.

Le rendement en acide trifluorométhanesulfinique est de 64 %.

Cette fraction est redistillée sous une pression de 25 mbar.

On obtient alors une première fraction (17 g) contenant 94 % massique d'eau et une seconde fraction (32 g) contenant 98 % en masse d'acide trifluorométhanesulfinique.

## Revendications

1. Procédé de préparation d'un acide trifluorométhanesulfinique de haute pureté, à partir d'un mélange aqueux comprenant un sel d'acide trifluorométhanesulfinique, un sel d'acide trifluoroacétique et des impuretés salines résultant de son procédé de préparation, **caractérisé par le fait que** ledit mélange est soumis aux opérations suivantes :
- acidification de telle sorte que l'on libère les sels de l'acide trifluoroacétique et de l'acide triflinique sous une forme acide,
- séparation des acides trifluoroacétique et trifluorométhanesulfinique par distillation permettant de récupérer l'acide trifluoroacétique en tête de distillation et l'acide trifluorométhanesulfinique en pied de distillation,
- séparation par distillation de l'acide trifluorométhanesulfinique présent dans le culot de distillation précédemment obtenu.

2. Procédé selon la revendication 1 **caractérisé par le fait que** le mélange aqueux est une solution aqueuse de 10 à 40 % en masse de matières sèches comprenant :
- de 5 à 35 % en masse d'un sel d'acide trifluorométhanesulfinique, de préférence un sel alcalin,
- de 5 à 35 % en masse d'un sel d'acide trifluoroacétique, de préférence un sel alcalin,
- de 0,5 à 2 % en masse d'impuretés salines.

3. Procédé selon la revendication 2 **caractérisé par le fait que** ladite solution aqueuse comprend de 15 à 20 % en masse d'un sel d'acide trifluorométhanesulfinique, de 10 à 15 % en masse d'un sel d'acide trifluoroacétique et de 0,5 à 2 % en masse d'impuretés salines.

4. Procédé selon l'une des revendications 1 à 3 **caractérisé par le fait que** l'on effectue une concentration du milieu réactionnel de façon à augmenter la concentration des sels des acides trifluoroacétique et triflinique de telle sorte qu'il y ait présence entre 2 et 60 % en masse, de préférence entre 5 et 50 % en masse d'eau dans la solution concentrée obtenue.

5. Procédé selon l'une des revendications 1 à 4 **caractérisé par le fait que** l'on élimine de l'eau par distillation sous pression atmosphérique ou inférieure à la pression atmosphérique ou par injection d'un fluide vapeur ou gaz inerte.

6. Procédé selon la revendication 1 **caractérisé par le fait que** l'on effectue l'acidification à l'aide d'acide sulfurique, chlorhydrique ou phosphorique, un oléum ou de l'acide chlorhydrique gazeux.

7. Procédé selon la revendication 6 **caractérisé par le fait que** la quantité d'acide fort mis en oeuvre est telle que le rapport entre le nombre de moles d'acide exprimé en ions H⁺ et le nombre de moles des sels d'acide trifluoroacétique et d'acide triflinique varie entre 1 et 10, de préférence entre 1 et 4.

8. Procédé selon l'une des revendications 6 et 7 **caractérisé par le fait que** l'on fait appel à une solution d'acide fort concentrée.

9. Procédé selon l'une des revendications 1 à 8 **caractérisé par le fait que** l'on recueille en fin de traitement acide, une solution ou une suspension aqueuse comprenant l'acide trifluoroacétique, l'acide triflinique, l'acide fort en excès, les sels formés suite à l'acidification et les formes acides correspondant aux impuretés salines.

10. Procédé selon la revendication 9 **caractérisé par le fait que** la solution ou la suspension obtenue comprend de 4 à 26 % en masse d'acide trifluoroacétique, de 4 à 27 % en masse d'acide triflinique et de 5 à 60 % en masse d'eau.

11. Procédé selon la revendication 1 **caractérisé par le fait que** l'on ajoute dans la solution ou la suspension aqueuse obtenue suite à la concentration mais avant l'acidification, un composé susceptible de piéger l'acide fluorhydrique.

12. Procédé selon la revendication 1 **caractérisé par le fait que** l'on soumet la phase aqueuse obtenue suite à l'acidification à une opération de distillation permettant de récupérer en tête de distillation l'acide trifluoroacétique et éventuellement de l'eau et en pied de distillation l'acide triflinique accompagné de l'acide fort en excès, des sels des acides forts notamment ceux provenant de l'acidification et de l'eau.

13. Procédé selon la revendication 12 **caractérisé par le fait que** la distillation est conduite à une température dans le bouilleur comprise entre 60°C et 90°C sous une pression allant de 700 mbar à 50 mbar.

14. Procédé selon la revendication 12 **caractérisé par le fait que** l'on refroidit la phase gazeuse comprenant l'acide trifluoroacétique et éventuellement de l'eau et la transforme sous forme liquide, par refroidissement à une température comprise entre - 20°C et 20°C.

15. Procédé selon la revendication 1 **caractérisé par le fait que** l'on soumet le culot de distillation comprenant l'acide triflinique à une opération de distillation permettant de récupérer en tête de distillation l'acide triflinique et en pied de distillation un culot comprenant de l'acide triflinique résiduaire, les sels des acides forts, l'acide fort en excès utilisé à l'acidification et de l'eau.

16. Procédé selon la revendication 15 **caractérisé par le fait que** l'on effectue la distillation de l'acide triflinique à une température inférieure à 100°C, de préférence inférieure à 90°C et sous pression réduite choisie de préférence inférieure à 50 mbar et plus particulièrement entre 0,1 mbar et 30 mbar et encore plus préférentiellement entre 5 mbar et 30 mbar.

17. Procédé selon la revendication 16 **caractérisé par le fait que** l'on ajoute dans le culot de distillation comprenant l'acide triflinique, un tiers solvant choisi parmi les hydrocarbures aliphatiques de préférence, le décane, la décaline, les coupes pétrolières commercialisées sous la dénomination ISOPAR ® ; les hydrocarbures aromatiques de préférence le toluène, les xylènes, le mésitylène ; les hydrocarbures aromatiques halogénés de préférence, le monochlorobenzène, le dichlorobenzène ou leurs mélanges.

18. Procédé selon la revendication 16 **caractérisé par le fait que** l'on récupère en tête de distillation l'acide triflinique éventuellement accompagné d'un tiers solvant qui est liquéfié par refroidissement à une température comprise entre - 20°C et 20°C.

19. Procédé selon l'une des revendications 1 à 18 **caractérisé par le fait que** l'acide triflinique obtenu a une pureté supérieure ou égale à 95 %, de préférence supérieure ou égale à 98 % et comprise plus préférentiellement entre 99 et 99,5 %.

## Claims

1. Process for the preparation of a trifluoromethanesulphinic acid of high purity starting from an aqueous mixture comprising a trifluoromethanesulphinic acid salt, a trifluoroacetic acid salt and saline impurities resulting from its process of preparation, **characterized in that** said mixture is subjected to the following operations:
- acidification in such a way that the salts of the trifluoroacetic acid and of the triflinic acid are released in an acid form,
- separation of the trifluoroacetic acid and the trifluoromethanesulphinic acid by distillation, making it possible to recover the trifluoroacetic acid at the distillation top and the trifluoromethanesulphinic acid at the distillation bottom,
- separation, by distillation, of the trifluoromethanesulphinic acid present in the distillation concentrate obtained above.

2. Process according to Claim 1, **characterized in that** the aqueous mixture is an aqueous solution having a solids content of 10 to 40% by weight comprising:
- from 5 to 35% by weight of a trifluoromethanesulphinic acid salt, preferably an alkali metal salt,
- from 5 to 35% by weight of a trifluoroacetic acid salt, preferably an alkali metal salt,
- from 0.5 to 2% by weight of saline impurities.

3. Process according to Claim 2, **characterized in that** said aqueous solution comprises from 15 to 20% by weight of a trifluoromethanesulphinic acid salt, from 10 to 15% by weight of a trifluoroacetic acid salt and from 0.5% to 2% by weight of saline impurities.

4. Process according to one of Claims 1 to 3, **characterized in that** the reaction medium is concentrated so as to increase the concentration of the salts of the trifluoroacetic acid and the triflinic acid in such a way that between 2 and 60% by weight, preferably between 5 and 50% by weight, of water is present in the concentrated solution obtained.

5. Process according to one of Claims 1 to 4, **characterized in that** water is removed by distillation at atmospheric pressure or under a pressure lower than atmospheric pressure or by injection of a steam or inert gas fluid.

6. Process according to Claim 1, **characterized in that** the acidification is carried out using sulfuric, hydrochloric or phosphoric acid, an oleum or gaseous hydrochloric acid.

7. Process according to Claim 6, **characterized in that** the amount of strong acid employed is such that the ratio of the number of moles of acid, expressed as H⁺ ions, to the number of moles of the salts of trifluoroacetic acid and of triflinic acid varies between 1 and 10, preferably between 1 and 4.

8. Process according to either of Claims 6 and 7, **characterized in that** a concentrated solution of strong acid is resorted to.

9. Process according to one of Claims 1 to 8, **characterized in that**, at the end of the acid treatment, an aqueous solution or suspension is collected which comprises the trifluoroacetic acid, the triflinic acid, the excess strong acid, the salts formed subsequent to the acidification and the acid forms corresponding to the saline impurities.

10. Process according to Claim 9, **characterized in that** the solution or suspension obtained comprises from 4 to 26% by weight of trifluoroacetic acid, from 4 to 27% by weight of triflinic acid and from 5 to 60% by weight of water.

11. Process according to Claim 1, **characterized in that** a compound capable of trapping hydrofluoric acid is added to the aqueous solution or suspension obtained, subsequent to the concentration but before the acidification.

12. Process according to Claim 1, **characterized in that** the aqueous phase obtained subsequent to the acidification is subjected to a distillation operation which makes it possible to recover, at the distillation top, the trifluoroacetic acid and optionally water and, at the distillation bottom, the triflinic acid, accompanied by the excess strong acid, the salts of the strong acids, in particular those originating from the acidification, and water.

13. Process according to Claim 12, **characterized in that** the distillation is carried out at a temperature in the reboiler of between 60°C and 90°C under a pressure ranging from 700 mbar to 50 mbar.

14. Process according to Claim 12, **characterized in that** the gas phase comprising the trifluoroacetic acid and optionally water is cooled and converted to the liquid form by cooling to a temperature of between -20°C and 20°C.

15. Process according to Claim 1, **characterized in that** the distillation concentrate comprising the triflinic acid is subjected to a distillation operation which makes it possible to recover, at the distillation top, the triflinic acid and, at the distillation bottom, a concentrate comprising residual triflinic acid, the salts of the strong acids, the excess strong acid used in the acidification and water.

16. Process according to Claim 15, **characterized in that** the triflinic acid is distilled at a temperature below 100°C, preferably below 90°C, and under a reduced pressure preferably chosen below 50 mbar and more particularly between 0.1 mbar and 30 mbar and more preferably still between 5 mbar and 30 mbar.

17. Process according to Claim 16, **characterized in that** a third solvent, chosen from aliphatic hydrocarbons, preferably decane, decalin or petroleum fractions sold under the Isopar^{®} name; aromatic hydrocarbons, preferably toluene, xylenes or mesitylene; halogenated aromatic hydrocarbons, preferably monochlorobenzene, dichlorobenzene or their mixtures, is added to the distillation concentrate comprising the triflinic acid.

18. Process according to Claim 16, **characterized in that** the triflinic acid, optionally accompanied by a third solvent, is recovered at the distillation top and is liquefied by cooling to a temperature of between -20°C and 20°C.

19. Process according to one of Claims 1 to 18, **characterized in that** the triflinic acid obtained has a purity of greater than or equal to 95%, preferably of greater than or equal to 98% and more preferably of between 99 and 99.5%.

## Patentansprüche

1. Verfahren zur Herstellung von hochreiner Trifluormethansulfinsäure ausgehend von einer wässrigen Mischung, die ein Trifluormethansulfinsäuresalz, ein Trifluoressigsäuresalz und aus dem Herstellungsverfahren stammende Salzverunreinigungen umfasst, **dadurch gekennzeichnet, dass** man die Mischung den folgenden Arbeitsgängen unterwirft:
- Ansäuern derart, dass die Salze von Trifluoressigsäure und Trifluormethansulfinsäure in Säureform freigesetzt werden,
- Abtrennen der Trifluoressigsäure und Trifluormethansulfinsäure durch Destillation, wodurch die Trifluoressigsäure am Kopf der Destillation und die Trifluormethansulfinsäure am Sumpf der Destillation gewonnen werden kann,
- destillatives Abtrennen der in dem zuvor erhaltenen Destillationsrückstand vorliegenden Trifluormethansulfonsäure.

2. Verfahren nach Anspruch 1, **dadurch gekennzeichnet, dass** es sich bei der wässrigen Mischung um eine wässrige Lösung mit einem Feststoffgehalt von 10 bis 40 Gew.-% handelt, die
- 5 bis 35 Gew.-% eines Trifluormethansulfinsäuresalzes, vorzugsweise eines Alkalimetallsalzes,
- 5 bis 35 Gew.-% eines Trifluoressigsäuresalzes, vorzugsweise eines Alkalimetallsalzes,
- 0,5 bis 2 Gew.-% Salzverunreinigungen umfasst.

3. Verfahren nach Anspruch 2, **dadurch gekennzeichnet, dass** die wässrige Lösung 15 bis 20 Gew.-% eines Trifluormethansulfinsäuresalzes, 10 bis 15 Gew.-% eines Trifluoressigsäuresalzes und 0,5 bis 2 Gew.-% Salzverunreinigungen umfasst.

4. Verfahren nach einem der Ansprüche 1 bis 3, **dadurch gekennzeichnet, dass** man das Reaktionsmedium zur Erhöhung der Konzentration der Salze von Trifluoressigsäure und Trifluormethansulfinsäure derart aufkonzentriert, dass in der erhaltenen konzentrierten Lösung zwischen 2 und 60 Gew.-%, vorzugsweise zwischen 5 und 50 Gew.-% Wasser vorliegen.

5. Verfahren nach einem der Ansprüche 1 bis 4, **dadurch gekennzeichnet, dass** man Wasser durch Destillation unter Normaldruck oder einem darunter liegenden Druck oder durch Einleiten eines Dampf- oder Inertgasfluids entfernt.

6. Verfahren nach Anspruch 1, **dadurch gekennzeichnet, dass** man das Ansäuern mit Hilfe von Schwefelsäure, Salzsäure oder Phosphorsäure, Oleum oder gasförmiger Salzsäure durchführt.

7. Verfahren nach Anspruch 6, **dadurch gekennzeichnet, dass** die eingesetzte Menge an starker Säure derart beschaffen ist, dass das Verhältnis zwischen der Zahl der Mole Säure, ausgedrückt als H⁺-Ionen, und der Zahl der Mole der Salze von Trifluoressigsäure und Trifluormethansulfinsäure zwischen 1 und 10, vorzugsweise zwischen 1 und 4, variiert.

8. Verfahren nach einem der Ansprüche 6 und 7, **dadurch gekennzeichnet, dass** man eine konzentrierte Lösung von starker Säure verwendet.

9. Verfahren nach einem der Ansprüche 1 bis 8, **dadurch gekennzeichnet, dass** man am Ende der Säurebehandlung eine wässrige Lösung oder Suspension, die die Trifluoressigsäure, die Trifluormethansulfinsäure, die überschüssige starke Säure, die nach dem Ansäuern gebildeten Salze und die den Salzverunreinigungen entsprechenden Säureformen umfasst, gewinnt.

10. Verfahren nach Anspruch 9, **dadurch gekennzeichnet, dass** die erhaltene Lösung oder Suspension 4 bis 26 Gew.-% Trifluoressigsäure, 4 bis 27 Gew.-% Trifluormethansulfinsäure und 5 bis 60 Gew.-% Wasser umfasst.

11. Verfahren nach Anspruch 1, **dadurch gekennzeichnet, dass** man die erhaltene wässrige Lösung oder Suspension nach dem Aufkonzentrieren, aber vor dem Ansäuern mit einer zum Abfangen von Fluorwasserstoffsäure befähigten Verbindung versetzt.

12. Verfahren nach Anspruch 1, **dadurch gekennzeichnet, dass** man die nach dem Ansäuern erhaltene wässrige Phase einer Destillation unterwirft, wodurch am Kopf der Destillation die Trifluoressigsäure und gegebenenfalls Wasser und am Sumpf der Destillation die Trifluormethansulfinsäure zusammen mit der überschüssigen starken Säure, den Salzen der starken Säuren, insbesondere den beim Ansäuern angefallenen Salzen, und Wasser gewonnen werden können.

13. Verfahren nach Anspruch 12, **dadurch gekennzeichnet, dass** man die Destillation bei einer Verdampfertemperatur zwischen 60°C und 90°C unter einem Druck im Bereich von 700 mbar bis 50 mbar durchführt.

14. Verfahren nach Anspruch 12, **dadurch gekennzeichnet, dass** man die die Trifluoressigsäure und gegebenenfalls Wasser umfassende Gasphase durch Abkühlen auf eine Temperatur zwischen -20°C und 20°C abkühlt und in die flüssige Form überführt.

15. Verfahren nach Anspruch 1, **dadurch gekennzeichnet, dass** man den die Trifluormethansulfinsäure umfassenden Destillationsrückstand einer Destillation unterwirft, wodurch am Kopf der Destillation Trifluormethansulfinsäure und am Sumpf der Destillation ein Rückstand, der die restliche Trifluormethansulfinsäure, die Salze der starken Säuren, die beim Ansäuern verwendete überschüssige starke Säure und Wasser gewonnen werden können.

16. Verfahren nach Anspruch 15, **dadurch gekennzeichnet, dass** man die Destillation der Trifluormethansulfinsäure bei einer Temperatur von weniger als 100°C, vorzugsweise weniger als 90°C, und unter vermindertem Druck, vorzugsweise weniger als 50 mbar und spezieller zwischen 0,1 mbar und 30 mbar und noch weiter bevorzugt zwischen 5 mbar und 30 mbar durchführt.

17. Verfahren nach Anspruch 16, **dadurch gekennzeichnet, dass** man den die Trifluormethansulfinsäure umfassenden Destillationsrückstand mit einem dritten Lösungsmittel, ausgewählt aus aliphatischen Kohlenwasserstoffen, vorzugsweise Decan, Dekalin, unter der Bezeichnung ISOPAR® vertriebenen Erdölschnitten; aromatischen Kohlenwasserstoffen, vorzugsweise Toluol, Xylolen, Mesitylen; halogenierten aromatischen Kohlenwasserstoffen, vorzugsweise Monochlorbenzol, Dichlorbenzol oder Mischungen davon, versetzt.

18. Verfahren nach Anspruch 16, **dadurch gekennzeichnet, dass** man am Kopf der Destillation die Trifluormethansulfinsäure, gegebenenfalls zusammen mit einem dritten Lösungsmittel, gewinnt und durch Abkühlen auf eine Temperatur zwischen -20°C und 20°C verflüssigt.

19. Verfahren nach einem der Ansprüche 1 bis 18, **dadurch gekennzeichnet, dass** die erhaltene Trifluormethansulfinsäure eine Reinheit größer gleich 95%, vorzugsweise größer gleich 98% und weiter bevorzugt zwischen 99 und 99,5% aufweist.
